# EUROPEAN PATENT SPECIFICATION

(11) **EP 1 196 083 B1**
(45) Date of publication and mention of the grant of the patent: **19.03.2008**
(21) Application number: 00944946.3
(22) Date of filing: 28.06.2000
(51) Int. Cl.: A61K 51/04, A61B 5/00, A61B 5/16

(54) **IMAGING THE DOPAMINE TRANSPORTER TO DETERMINE AD-HD**
ABBILDEN DER DOPAMINTRANSPORTER ZUR BESTIMMUNG DES AD-HD
IMAGERIE DU TRANSPORTEUR DE DOPAMINE AFIN DE DETERMINER TDAH

(30) Priority: 28.06.1999 US 141540 P
(43) Date of publication of application: 17.04.2002
(73) Proprietor: President and Fellows of Harvard College, Cambridge, MA 02138 (US); THE GENERAL HOSPITAL CORPORATION, Boston, MA 02114 (US); ORGANIX, INC., Woburn, MA 01801 (US)
(72) Inventor: MADRAS, Bertha, K., Newton, MA 02458 (US); FISCHMAN, Alan, J., Boston, MA 02114 (US); MELTZER, Peter, C., Lexington, MA 02173 (US)
(74) Representative: Hofer, Dorothea
(86) International application number: PCT/US2000/017769
(87) International publication number: WO 2001/000082

(56) References cited:
- WO-A-97/16210
- US-A- 5 770 180
- US-A- 6 011 070
- DATABASE BIOSIS [Online] BIOSCIENCES INFORMATION SERVICE, PHILADELPHIA, PA, US; December 1998 (1998-12) WALDMAN I D ET AL.: "Association and linkage of the dopamine transporter gene and attention-deficit hyperactivity disorder in children: Heterogeneity owing to diagnostic subtype and severity." Database accession no. PREV199900299972 XP002239035
- SWANSON J M ET AL: "Attention-deficit hyperactivity disorder and hyperkinetic disorder" LANCET, XX, XX, vol. 351, no. 9100, 7 February 1998 (1998-02-07), pages 438-440, XP004265114 ISSN: 0140-6736
- DATABASE MEDLINE [Online] US NATIONAL LIBRARY OF MEDICINE (NLM), BETHESDA, MD, US; June 1998 (1998-06) FISCHMAN ET AL.: "Rapid detection of Parkinson's disease by SPECT with altropane: a selective ligand for dopamine transporters." Database accession no. NML9593103 XP002239036
- DOUGHERTY D D ET AL: "Dopamine transporter density in patients with attention deficit hyperactivity disorder" LANCET, XX, XX, vol. 354, no. 9196, 18 December 1999 (1999-12-18), pages 2132-2133, XP004263271 ISSN: 0140-6736
- KRAUSE K. H. ET AL.: "Increased striatal dopamine transporter in adult patients with attention deficit hyperactivity disorder: effects of methylphenidate as measured by single photon emission computed tomography" NEUROSCIENCE LETTERS, vol. 285, no. 2, 12 May 2000 (2000-05-12), pages 107-110, XP002239034

## Description

### Background of the Invention

Attention deficit-hyperactivity disorder (AD-HD) is a recognized syndrome characterized by a relatively high (2-6%) incidence in children with persistence into adulthood. Some research suggests that AD-HD has a genetic component. Biederman et al., "High Risk for Attention Deficit Disorder Among Children of Parents with Childhood Onset of the Disorder: A Pilot Study," Am. J. Psychiatry, 152(3):431-35 (1995); Arnold et al., "National Institute of Health Collaborative Multimodal Treatment Study of Children with ADHD (the MTA)," Arch. Gen. Psychiatry, 54:865-70 (1997). Paradoxically, stimulant drugs such as methylphenidate, d-amphetamine and pemoline are effective medications for treating AD-HD in children and adults. Seeman and Madras, "Anti-hyperactivity Medication: Methylphenidate and amphetamine," Molecular Psychiatry, 3:385-96 (1998); Arnold et al., *supra*; Greenhill et al., "Stimulant Medications," J. Am. Acad. Child Adolesc. Psychiatry, 38(5):503-12 (1999); Wilens and Biederman, "The Stimulants," Psychiatric Clinics of N. Am., 15(1): 191-222 (1992).

Increased recognition of the disorder has led to increased prescription of stimulant medications for treating AD-HD. There is concern about the possibility of over-diagnosis of AD-HD and resulting unnecessary treatment with stimulant drugs that have inherent potential for abuse. Conversely, if AD-HD is underdiagnosed, patients who could be helped may go untreated.

### Summary of the Invention

We have recognized that the dopamine transporter in the human brain is a useful protein for diagnosing and monitoring the course of AD-HD. For example, various dopamine transporter imaging agents can be used to assay the dopamine transporter as a biological marker for AD-HD. Such imaging is used to diagnose AD-HD and to monitor it, e.g. as the patient matures and/or is treated over time.

Thus, the present invention provides methods according to claims 1, 18, 27 and 30. Preferred embodiments are set forth in sub-claims.

In general, in one aspect, the invention features methods of diagnosing attention deficient-hyperactivity disorder (AD-HD) in a human patient by assessing dopamine transporter in at least one region of said patient's central nervous system. An elevated level of dopamine transporter in said patient is indicative of AD-HD. This assessment can involve an assessment of the binding of a dopamine transporter ligand to dopamine transporter. PET or SPECT imaging are particularly preferred assessment techniques. Suitable ligands include [¹¹C]CFT ([¹¹C]WIN 35,428), [¹²³I]altropane, and [¹⁸F]CFT. Other suitable ligands, particularly for PET, include [¹¹C]altropane. Other suitable ligands, particularly for SPECT, include technetium-labeled phenyltropane probes, such as [99mTc]technepine, O-1505, and similar compounds. The portion of the patient's central nervous system is preferably a portion of the human brain, e.g., the striatum.

Assessing dopamine transporter to determine dopamine transporter levels may include assessing dopamine transporter availability or binding potential.

In general, in another aspect, the invention features a method of determining the effectiveness of an AD-HD treatment for a human patient. The method includes assessing an initial dopamine transporter level in at least one region of the patient's central nervous system, applying the treatment, and then assessing a subsequent dopamine transporter level in the region, e.g., more than two weeks later. The initial and subsequent dopamine transporter levels are then compared.

In this aspect of the invention, the treatment can include administration of a pharmaceutical, such as methylphenidate, pemoline, or an amphetamine, and the assessing step can include PET or SPECT.

In general, in another aspect, the invention features a method of determining whether an individual has a heightened probability of having AD-HD. The method includes assessing dopamine transporter in at least one region of the patient's central nervous system and comparing the patient's dopamine transporter level to a normal dopamine transporter level. A higher than normal level is indicative of a heightened probability of having AD-HD.

In general, in another aspect, the invention features a method of monitoring the progress of a treatment for AD-HD in a human patient. The method includes assessing dopamine transporter in at least one region of the patient's central nervous system a plurality of times during the treatment.

The invention may include one or more of the following advantages. Assessing dopamine transporter levels allows an objective, biologically based diagnosis of AD-HD. Diagnosis based on dopamine transporter levels can be used for patients of all ages and both sexes.

The imaging agents used to assess dopamine transporter levels, such as [¹²³I]altropane, are safe and well tolerated by patients.

Other features and advantages of the invention will be apparent to those skilled in the arts from the following description of the preferred embodiments and from the claims.

### Description of the Preferred Embodiments

We have discovered that abnormal levels of the dopamine transporter in human brain is indicative of AD-HD. Assessing dopamine levels in the brain, therefore, can confirm a diagnosis of AD-HD. or can assist in monitoring treatment of AD-HD.

### Dopamine Transporter Assessment Techniques

Assessing dopamine transporter levels can be performed by assessing dopamine transporter availability using, e.g., PET (positron emission tomography) or SPECT (single photon emission computed tomography). To measure dopamine transporter availability, a labeled probe that targets the transporter is introduced into the brain, e.g.. intravenously, and PET or SPECT is performed. From the PET or SPECT images, the density of the dopamine transporter is quantified by measuring the binding potential, where binding potential is defined as the maximum number of binding cites, Bₘₐₓ, divided by a dissociation constant, K_{d}, where K_{d} is related to affinity.

Imaging agents that target the dopamine transporter include [¹¹C]altropane, [¹¹C or ¹⁸F]WIN 35,428 ([¹¹C]CFT), [¹²³I]altropane, [99^{m}Tc]O-1505, [99^{m}Tc]technepine, and similar compounds. These agents bind the dopamine transporter with varying affinities, allowing multiple, dissimilar assessments to be performed. Structures, synthesis, and/or sources of some of the above agents are described in, e.g., Fischman et al., "Rapid Detection of Parkinson's Disease by SPECT with Altropane: A Selective Ligand For Dopamine Transporters." Synapse, 29:125-41 (1998) ([¹²³I]altropane); Madras et al., "Technipine: A High Affinity 99mTechnetium Probe to Label the Dopamine Transporter in Brain by SPECT Imaging," Synapse, 22:239-46 (1996); Meltzer et al., "Substituted 3-Phenyltropane Analogs of Cocaine: Synthesis, Inhibition of Binding of Cocaine Recognition Sites, and Positron Emission Tomography Imaging," J. Med. Chem., 36:855-62 (1993); and Milius et al., "Synthesis and Receptor Binding of N-Substituted Tropine Derivatives, High Affinity Ligands for the Cocaine Receptor," J. Medicinal Chem., 34:1728-31 (1990), each of which is incorporated by reference herein in its entirety.

Other techniques to identify anomalies in brains of AD-HD patients include SPECT imaging to measure blood flow and functional MRI to measure regional metabolic function. Both techniques demonstrate abnormal function of the frontal cortex.

### Applications of Dopamine Transporter Assessments

Traditionally, AD-HD is diagnosed by assessing a patient's cognitive and attentional skills. For example, according to the Diagnostic and Statistical Manual of Mental Disorders, 4th edition (DSM-IV), evidence of either inattention or hyperactivity and impulsivity are required to support a diagnosis of AD-HD. Under the DSM-IV standard, a finding of inattention must be supported by six or more of the following symptoms, persisting for at least six months to a degree that is maladaptive and inconsistent with the patient's level of development: (1) fails to give close attention to details; (2) has difficulty sustaining attention to activities: (3) does not listen when spoken to directly; (4) does not follow through on instructions: (5) has difficulty organizing tasks; (6) avoids engaging in tasks that require sustained mental effort; (7) loses things necessary for activities; (8) is easily distracted by extraneous stimuli; (9) is forgetful in daily activities. Similarly, under the DSM-IV standard, a finding of hyperactivity and impulsivity must be supported by six or more of the following symptoms, persisting for at least six months to a degree that is maladaptive and inconsistent with the patient's level of development: (1) is fidgety; (2) leaves seat when expected to remain seated: (3) runs about in situations in which it is inappropriate; (4) has difficulty playing quietly; (5) acts as if "driven by a motor"; (6) talks excessively; (7) blurts out answers before questions have been completed; (8) has difficulty taking turns; (9) interrupts or intrudes on others. When used to diagnose AD-HD in adults, the above criteria are modified slightly to apply to adult environments.

Diagnosis of AD-HD using the DSM-IV standard is inherently somewhat subjective, leading to inconsistent diagnosis.

Assessment of dopamine transporter levels can complement, and in some cases, supplant, traditional AD-HD diagnostic techniques. The dopamine transporter level assessments using PET or SPECT provide objective, biological criteria for diagnosing AD-HD, and can be used, e.g., to confirm an AD-HD diagnosis under the DSM-IV standard, to resolve conflicting diagnoses, or to call into question a diagnosis or non-diagnosis of AD-HD. Dopamine transport assessments can also be used to refine subjective testing criteria for AD-HD.

In addition. PET and SPECT imaging of the dopamine transporter can be used to monitor and adjust treatment of AD-HD. For example, methylphenidate, a drug commonly used to treat AD-HD, may occupy the dopamine transporter. Volkow et al., "Dopamine Transporter Occupancies in the Human Brain Induced by Therapeutic Doses of Oral Methylphenidate," Am J Psychiatry, 155:1325-31 (1998). The effectiveness of methylphenidate treatment for a particular patient could be monitored by assessing dopamine transporter levels both before and after administration of methylphenidate. For example, dopamine transporter levels can be assessed immediately before a treatment, and then, e.g., two weeks, months, or longer after administration of treatment. If the methylphenidate successfully occupies dopamine transporters, it will displace the radio-labeled probe, decreasing the availability of dopamine transporter. The decreased availability of dopamine transporter will manifest as decreased binding potential in the PET or SPECT images. Such objective data can assist a physician in determining the most effective drug and the most effective dosage for a particular patient.

Dopamine transporter level assessments can also be used to monitor treatment over the long term, and to help a physician and patient determine whether treatment affects transporter levels and whether treatment can be stopped.

Finally, dopamine transporter level assessments can identify individuals at risk for AD-HD. Patients found to have elevated dopamine transporter levels can, e.g.. be referred for conventional AD-HD testing.

### Experimental Studies

Experimental data confirm the efficacy of diagnosing AD-HD by detecting elevated dopamine transporter levels.

SPECT imaging of the dopamine transporter with [¹²³I]altropane was conducted on six subjects previously diagnosed with AD-HD, and on control individuals without a diagnosis of AD-HD. For each individual tested, greater than I mCi of [¹²³I]altropane was administered by intravenous injection at the onset of imaging. Images of the striatum were collected and analyzed by a radiologist to determine striatal binding potentials. In general, the methodology used for the SPECT imaging was the same as the methods described in Fischman et al., "Rapid Detection of Parkinson's Disease by SPECT with Altropane: A Selective Ligand For Dopamine Transporters." Synapse, 29:125-41 (1998), which is incorporated herein by reference in its entirety.

The results of the imaging of AD-HD patients are summarized below in Table 1:

**Table 1: AD-HD Patients**

| INDIVIDUAL | AGE | BINDING POTENTIAL |
|---|---|---|
| ADHD-1 | 34 | 2.98 |
| ADHD-2 | 45 | 2.34 |
| ADHD-3 | 24 | 2.71 |
| ADHD-4 | 53 | 2.33 |
| ADHD-5 | 51 | 2.16 |
| ADHD-6 | 41 | 3.51 |
| MEAN | 41 | 2.67 |

These six AD-HD individuals show elevated binding potential, and, therefore, elevated dopamine transporter levels compared to expected levels for aged matched normal individuals, which may range, generally, between about 1.0 and 2.2.

## Claims

1. A method of diagnosing attention deficient-hyperactivity disorder (AD-HD) in a human patient comprising
- providing a dopamine transporter imaging agent;
- introducing said dopamine transporter imaging agent into the brain, and
- assessing whether dopamine transporter in at least one region of said patient's central nervous system is elevated.

2. The method of claim 1 wherein said assessment is by PET or SPECT imaging.

3. The method of claim 1, further comprising administering to the patient a labeled dopamine transporter ligand and wherein the assessment comprises determining the amount of labeled dopamine transporter ligand that is bound to dopamine transporter.

4. The method of claim 3, whcrcin the method further comprises the step of comparing the amount of labeled dopamine transporter ligand that is bound to dopamine transporter with a control.

5. The method of claim 3, wherein the dopamine transporter ligand comprises a compound that binds to the dopamine transporter.

6. The method of claim 3, wherein said dopamine transporter ligand comprises [¹¹C]CFT ([¹¹C]WIN 35,428).

7. The method of claim 6, wherein said assessment comprises imaging by PET.

8. The method of claim 3, wherein said dopamine transporter ligand comprises [¹¹C]altropane.

9. The method of claim 8, wherein said assessment comprises imaging by PET.

10. The method of claim 3, wherein said ligand comprises [¹²³I]altropane and said assessment comprises imaging by SPECT.

11. The method of claim 3, wherein, said ligand comprises a technetium-labeled phenyltropane probe.

12. The method of claim 11, wherein said ligand comprises [⁹⁹mTc]technepine, O-1505.

13. The method of claim 12, wherein said assessment comprises imaging by SPECT.

14. The method of claim 1, wherein said at least one region of said patient's central nervous system comprises a portion of the brain.

15. The method of claim 14, wherein said portion of the brain comprise the striatum.

16. The method of claim 1, further comprising administering to the patient a labeled dopamine transporter ligand, the assessing step comprises assessing dopamine transporter availability, and the method further comprises comparing dopamine transporter availability with the dopamine transporter availability in a control, wherein a higher dopamine transporter availability in said patient is indicative of ADHD.

17. The method of claim 1, further comprising administering to the patient a labeled dopamine transporter ligand, the assessing step comprises determining dopamine transporter binding potential and the method further comprises comparing dopamine transporter binding potential with the dopamine transporter binding potential in a control, wherein a higher dopamine transporter binding potential in said patient is indicative of ADHD.

18. A method of determining the effectiveness of an AD-HD treatment for a human patient, the method comprising:
- providing a dopamine transporter imaging agent;
- by using said dopamine transporter imaging agent:
a) assessing an initial dopamine transporter level in at least one region of said patient's central nervous system and,
b) after applying the treatment,
c) assessing a subsequent dopamine transporter level in said at least one region of said patient's central nervous system; and
d) comparing the initial and subsequent dopamine transporter levels.

19. The method of claim 18, further comprising administering to the patient a labeled dopamine transporter ligand before at least one of the assessing steps, and wherein the assessing comprises determining the amount of labeled dopamine transporter ligand that is bound to dopamine transporter.

20. The method of claim 19, wherein the dopamine transporter ligand comprises a compound that binds to the dopamine transporter.

21. The method of claim 18, wherein the second assessing step occurs two weeks or more after the applying step.

22. The method of claim 18, wherein the treatment comprises a pharmaceutical treatment.

23. The method of claim 22, wherein the pharmaceutical treatment comprises administration of a pharmaceutical selected from the group consisting of methylphenidate, pemoline, and an amphetamine.

24. The method of claim 18, wherein the assessing step comprises PET or SPECT imaging.

25. The method of claim 18, further comprising administering to the patient a labeled dopamine transporter ligand before at least one of the assessing steps, the assessing steps comprise assessing dopamine transporter availability, and the comparing step comprises comparing dopamine transporter availability in step (a) with the dopamine transporter availability in step (c), wherein a lower dopamine transporter availability in step (c) indicates that the treatment is effective.

26. The method of claim 18, further comprising administering to the patient a labeled dopamine transporter ligand before at least one of the assessing steps, the assessing steps comprise assessing dopamine transporter binding potential, and the comparing step comprises comparing dopamine transporter binding potential in step (a) with the dopamine transporter binding potential in step (c), wherein a lower dopamine transporter binding potential in step (c) indicates that the treatment is effective.

27. A method of determining whether an individual has a heightened probability of having AD-HD, the method comprising:
- providing a dopamine transporter imaging agent;
- assessing dopamine transporter in at least one region of said patient's central nervous system by using said dopamine transporter imaging agent;
- comparing said patient's dopamine transporter level to a normal dopamine transporter level, wherein a higher than normal level indicates a heightened probability of having AD-HD.

28. The method of claim 27, further comprising administering to the patient a labeled dopamine transporter ligand before the assessing step, and wherein the assessing comprises determining the amount of labeled dopamine transporter ligand that is bound to dopamine transporter.

29. The method of claim 28, wherein the dopamine transporter ligand comprises a compound that binds to the dopamine transporter.

30. A method of monitoring the progress of a treatment for AD-HD in a human patient, the method comprising
- providing a dopamine transporter imaging agent;
- by using said dopamine transporter imaging agent assessing dopamine transporter in at least one region of said patient's central nervous system a plurality of times during said treatment.

31. The method of claim 30, further comprising administering to the patient a labeled dopamine transporter ligand before the assessing step, and wherein the assessing comprises determining the amount of labeled dopamine transporter ligand that is bound to dopamine transporter.

32. The method of claim 31, wherein the dopamine transporter ligand comprises a compound that binds to the dopamine-transporter.

33. The method of claim 30, wherein the treatment comprises a pharmaceutical treatment.

34. The method of claim 33, wherein the pharmaceutical treatment comprises administration of a pharmaceutical selected from the group consisting of methylphenidate, pemoline, and an amphetamine.

35. The method of claim 34, wherein the assessing step comprises PET or SPECT imaging.

36. The method according to any one of claims 1, 16, 17, 18, 19, 21, 25, 26, 27, 28, 30 and 31, wherein the assessment is non-invasive to the human body.

## Patentansprüche

1. Verfahren zur Diagnose von Aufmerksamkeitsdefizit-Hyperaktivitätssyndrom (AD-HS) in einem Humanpatienten, umfassend:
- Bereitstellen eines Dopamintransporter-bildgebenden Mittels,
- Einbringen des Dopamintransporter-bildgebenden Mittels in das Gehirn, und
- Beurteilen, ob der Dopamintransporter in mindestens einem Bereich des zentralen Nervensystems des Patienten erhöht ist.

2. Verfahren von Anspruch 1, wobei die Beurteilung durch PET- oder SPECT-Bildgebung erfolgt.

3. Verfahren von Anspruch 1, ferner umfassend die Verabreichung - gegenüber dem Patienten - eines markierten Dopamintransporter-Liganden, und wobei die Beurteilung die Bestimmung der Menge des markierten Dopamintransporter-Liganden umfasst, der an den Dopamintransporter gebunden ist.

4. Verfahren von Anspruch 3, wobei das Verfahren ferner den Schritt des Vergleichens der Menge des markierten Dopamintransporter-Liganden, der an den Dopamintransporter gebunden ist, mit einer Kontrolle umfasst.

5. Verfahren von Anspruch 3, wobei der Dopamintransporter-Ligand eine Verbindung umfasst, die an den Dopamintransporter bindet.

6. Verfahren von Anspruch 3, wobei der Dopamintransporter-Ligand [¹¹C]CFT ([¹¹C]WIN 35,428) umfasst.

7. Verfahren von Anspruch 6, wobei die Beurteilung eine Bildgebung durch PET umfasst.

8. Verfahren von Anspruch 3, wobei der Dopamintransporter-Ligand [¹¹C]-Altropan umfasst.

9. Verfahren von Anspruch 8, wobei die Beurteilung eine Bildgebung durch PET umfasst.

10. Verfahren von Anspruch 3, wobei der Ligand [¹²³I]-Altropan umfasst und die Beurteilung eine Bildgebung durch SPECT umfasst.

11. Verfahren von Anspruch 3, wobei der Ligand eine Technetium-markierte Phenyltropan-Sonde umfasst.

12. Verfahren von Anspruch 11, wobei der Ligand [⁹⁹mTc]-Technepin, O-1505, umfasst.

13. Verfahren von Anspruch 12, wobei die Begutachtung eine Bildgebung durch SPECT umfasst.

14. Verfahren von Anspruch 1, wobei mindestens ein Bereich des zentralen Nervensystems des Patienten ein Bereich des Gehirns umfasst.

15. Verfahren von Anspruch 14, wobei der Bereich des Gehirns das Striatum umfasst.

16. Verfahren von Anspruch 1, ferner umfassend die Verabreichung - gegenüber dem Patienten - eines markierten Dopamintransporter-Liganden, wobei der Schritt der Begutachtung die Begutachtung der Dopamintransporter-Verfügbarkeit umfasst, und wobei das Verfahren ferner das Vergleichen der Dopamintransporter-Verfügbarkeit mit der Dopamintransporter-Verfügbarkeit in einer Kontrolle umfasst, wobei eine höhere Dopamintransporter-Verfügbarkeit im Patienten auf ADHD hinweist.

17. Verfahren von Anspruch 1, ferner umfassend die Verabreichung - gegenüber dem Patienten - eines markierten Dopamintransporter-Liganden, wobei der Schritt der Begutachtung die Bestimmung des Dopamintransporter-Bindepotentials umfasst, und wobei das Verfahren ferner den Vergleich des Dopamintransporter-Bindepotentials mit dem Dopamintransporter-Bindepotential in einer Kontrolle umfasst, wobei ein höheres Dopamintransporter-Bindepotential im Patienten auf ADHD hinweist.

18. Verfahren zum Bestimmen der Wirksamkeit einer AD-HD-Behandlung für einen Humanpatienten, wobei das Verfahren umfasst:
- Bereitstellen eines Dopamintransporter-bildgebenden Mittels;
- durch Verwendung des Dopamintransporter-bildgebenden Mittels:
a) Begutachten eines anfänglichen Dopamintransporter-Niveaus in mindestens einem Bereich des zentralen Nervensystems des Patienten, und
b) nach Anwenden der Behandlung
c) Begutachten eines nachfolgenden Dopamintransporter-Niveaus in dem mindestens einen Bereich des zentralen Nervensystems des Patienten; und
d) Vergleich der anfänglichen und nachfolgenden Dopamintransporter-Niveaus.

19. Verfahren von Anspruch 18, ferner umfassend das Verabreichen - gegenüber dem Patienten - eines Dopamintransporter-Liganden vor mindestens einem der Begutachtungsschritte, und wobei die Begutachtung die Bestimmung der Menge des markierten Dopamintransporter-Liganden, der am Dopamintransporter gebunden ist, umfasst.

20. Verfahren von Anspruch 19, wobei der Dopamintransporter-Ligand eine Verbindung umfasst, die an den Dopamintransporter bindet.

21. Verfahren von Anspruch 18, wobei der zweite Begutachtungsschritt zwei Wochen oder mehr nach dem Anwendungsschritt abläuft.

22. Verfahren von Anspruch 18, wobei die Behandlung eine pharmazeutische Behandlung umfasst.

23. Verfahren von Anspruch 22, wobei die pharmazeutische Behandlung die Verabreichung eines Pharmazeutikums umfasst, das aus der aus Methylphenidat, Pemolin und Amphetamin bestehenden Gruppe ausgewählt ist.

24. Verfahren von Anspruch 18, wobei der Begutachtungsschritt eine PET- oder SPECT-Bildgebung umfasst.

25. Verfahren von Anspruch 18, ferner umfassend das Verabreichen - gegenüber dem Patienten - eines markierten Dopamintransporter-Liganden vor mindestens einem der Begutachtungsschritte, wobei die Begutachtungsschritte das Begutachten der Dopamintransporter-Verfügbarkeit umfasst, und wobei der Vergleichsschritt den Vergleich der Dopamintransporter-Verfügbarkeit im Schritt (a) mit der Dopamintransporter-Verfügbarkeit im Schritt (c) umfasst, wobei eine niedrigere Dopamintransporter-Verfügbarkeit im Schritt (c) darauf hinweist, dass die Behandlung wirksam ist.

26. Verfahren von Anspruch 18, ferner umfassend das Verabreichen - gegenüber dem Patienten - eines markierten Dopamintransporter-Liganden vor mindestens einem der Begutachtungsschritte, wobei die Begutachtungsschritte das Begutachten des Dopamintransporter-Bindepotentials umfasst, und wobei der Vergleichsschritt den Vergleich des Dopamintransporter-Bindepotentials in Schritt (a) mit dem Dopamintransporter-Bindepotential in Schritt (c) umfasst, wobei ein niedrigeres Dopamintransporter-Bindepotential in Schritt (c) darauf hinweist, dass die Behandlung wirksam ist.

27. Verfahren zum Bestimmen, ob ein Individuum eine erhöhte Wahrscheinlichkeit besitzt, AD-HD zu haben, wobei das Verfahren umfasst:
- Bereitstellen eines Dopamintransporter-bildgebenden Mittels;
- Begutachten des Dopamintransporters in mindestens einem Bereich des zentralen Nervensystems des Patienten durch Verwenden des Dopamintransporter-bildgebenden Mittels;
- Vergleichen des Dopamintransporter-Niveaus des Patienten gegenüber einem normalen Dopamintransporter-Niveau, wobei ein höheres Niveau als normal auf eine erhöhte Wahrscheinlichkeit hinweist, AD-HD zu haben.

28. Verfahren von Anspruch 27, ferner umfassend das Verabreichen - gegenüber dem Patienten - eines markierten Dopamintransporter-Liganden vor dem Begutachtungsschritt, und wobei die Begutachtung die Bestimmung der Menge des markierten Dopamintransporter-Liganden, der an den Dopamintransporter gebunden ist, umfasst.

29. Verfahren von Anspruch 28, wobei der Dopamintransporter-Ligand eine Verbindung umfasst, die an den Dopamintransporter bindet.

30. Verfahren zur Beobachtung des Verlaufs einer Behandlung für AD-HD in einem Humanpatienten, wobei das Verfahren umfasst:
- Bereitstellen eines Dopamintransporter-bildgebenden Mittels;
- durch Verwendung des Dopamintransporter-bildgebenden Mittels: Begutachten des Dopamintransporters in mindestens einem Bereich des zentralen Nervensystems des Patienten mehrfach während der Behandlung.

31. Verfahren von Anspruch 30, ferner umfassend das Verabreichen - gegenüber dem Patienten - eines markierten Dopamintransporter-Liganden vor dem Begutachtungsschritt, und wobei die Begutachtung die Bestimmung der Menge des markierten Dopamintransporter-Liganden, der an den Dopamintransporter gebunden ist, umfasst.

32. Verfahren von Anspruch 31, wobei der Dopamintransporter-Ligand eine Verbindung umfasst, die an den Dopamintransporter bindet.

33. Verfahren von Anspruch 30, wobei die Behandlung eine pharmazeutische Behandlung umfasst.

34. Verfahren von Anspruch 33, wobei die pharmazeutische Behandlung das Verabreichen eines Pharmazeutikums umfasst, der aus der aus Methylphenidat, Pemolin und einem Amphetamin bestehenden Gruppe ausgewählt ist.

35. Verfahren von Anspruch 34, wobei der Begutachtungsschritt eine PET- oder SPECT-Bildgebung umfasst.

36. Verfahren gemäß irgendeinem der Ansprüche 1, 16, 17, 18, 19, 21, 25, 26, 27, 28, 30 und 31, wobei die Begutachtung nicht-invasiv gegenüber dem menschlichen Körper ist.

## Revendications

1. Procédé de diagnostic d'un trouble déficitaire de l'attention avec hyperactivité (TDAH) chez un patient humain, comprenant :
- la fourniture d'un agent d'imagerie du transporteur de la dopamine ;
- l'introduction dudit agent d'imagerie du transporteur de la dopamine dans le cerveau, et
- l'évaluation du fait que le transporteur de la dopamine dans au moins une région du système nerveux central dudit patient est élevé.

2. Procédé selon la revendication 1, dans lequel ladite évaluation est réalisée par imagerie PET ou SPECT.

3. Procédé selon la revendication 1, comprenant en outre l'administration au patient d'un ligand marqué du transporteur de la dopamine et dans lequel l'évaluation comprend la détermination de la quantité de ligand marqué du transporteur de la dopamine qui est lié au transporteur de la dopamine.

4. Procédé selon la revendication 3, dans lequel le procédé comprend en outre l'étape consistant à comparer la quantité de ligand marqué du transporteur de la dopamine qui est lié au transporteur de la dopamine à un contrôle.

5. Procédé selon la revendication 3, dans lequel le ligand du transporteur de la dopamine comprend un composé qui se lie au transporteur de la dopamine.

6. Procédé selon la revendication 3, dans lequel ledit ligand du transporteur de la dopamine comprend [¹¹C]CFT ([¹¹C]WIN 35,428).

7. Procédé selon la revendication 6, dans lequel ladite évaluation comprend l'imagerie par PET.

8. Procédé selon la revendication 3, dans lequel ledit ligand du transporteur de la dopamine comprend le [¹¹C]altropane.

9. Procédé selon la revendication 8, dans lequel ladite évaluation comprend l'imagerie par PET.

10. Procédé selon la revendication 3, dans lequel ledit ligand comprend le [¹²³I]altropane et ladite évaluation comprend l'imagerie par SPECT.

11. Procédé selon la revendication 3, dans lequel ledit ligand comprend une sonde de phényltropane marquée au technétium.

12. Procédé selon la revendication 11, dans lequel ledit ligand comprend de la [⁹⁹mTc]technépine, O-1505.

13. Procédé selon la revendication 12, dans lequel ladite évaluation comprend l'imagerie par SPECT.

14. Procédé selon la revendication 1, dans lequel ladite au moins une région du système nerveux central dudit patient comprend une partie du cerveau.

15. Procédé selon la revendication 14, dans lequel ladite partie du cerveau comprend le striatum.

16. Procédé selon la revendication 1, comprenant en outre l'administration au patient d'un ligand marqué du transporteur de la dopamine, l'étape d'évaluation comprend l'évaluation de la disponibilité du transporteur de la dopamine, et le procédé comprend en outre la comparaison de la disponibilité du transporteur de la dopamine à la disponibilité du transporteur de la dopamine dans un contrôle, dans lequel une plus grande disponibilité du transporteur de la dopamine chez ledit patient est un indicateur de TDAH.

17. Procédé selon la revendication 1, comprenant en outre l'administration au patient d'un ligand marqué du transporteur de la dopamine, l'étape d'évaluation comprend la détermination du potentiel de liaison du transporteur de la dopamine et le procédé comprend en outre la comparaison du potentiel de liaison du transporteur de la dopamine au potentiel de liaison du transporteur de la dopamine dans un contrôle, dans lequel un plus grand potentiel de liaison du transporteur de la dopamine chez ledit patient est un indicateur de TDAH.

18. Procédé de détermination de l'efficacité d'un traitement d'un TDAH pour un patient humain, le procédé comprenant :
- la fourniture d'un agent d'imagerie du transporteur de la dopamine ;
- en utilisant ledit agent d'imagerie du transporteur de la dopamine :
a) l'évaluation d'un niveau initial de transporteur de la dopamine dans au moins une région du système nerveux central dudit patient, et
b) après l'application du traitement,
c) l'évaluation d'un niveau subséquent du transporteur de la dopamine dans ladite au moins une région du système nerveux central dudit patient ; et
d) la comparaison des niveaux initial et subséquent de transporteur de la dopamine.

19. Procédé selon la revendication 18, comprenant en outre l'administration au patient d'un ligand marqué du transporteur de la dopamine avant au moins une des étapes d'évaluation, et dans lequel l'évaluation comprend la détermination de la quantité de ligand marqué du transporteur de la dopamine qui est lié au transporteur de la dopamine.

20. Procédé selon la revendication 19, dans lequel le ligand du transporteur de la dopamine comprend un composé qui se lie au transporteur de la dopamine.

21. Procédé selon la revendication 18, dans lequel la deuxième étape d'évaluation a lieu deux semaines ou plus après l'étape d'application.

22. Procédé selon la revendication 18, dans lequel le traitement comprend un traitement pharmaceutique.

23. Procédé selon la revendication 22, dans lequel le traitement pharmaceutique comprend l'administration d'un produit pharmaceutique choisi dans le groupe constitué par le méthylphénidate, la pémoline, et une amphétamine.

24. Procédé selon la revendication 18, dans lequel l'étape d'évaluation comprend l'imagerie PET ou SPECT.

25. Procédé selon la revendication 18, comprenant en outre l'administration au patient d'un ligand marqué du transporteur de la dopamine avant au moins une des étapes d'évaluation, les étapes d'évaluation comprennent l'évaluation de la disponibilité du transporteur de la dopamine, et l'étape de comparaison comprend la comparaison de la disponibilité du transporteur de la dopamine dans l'étape (a) à la disponibilité du transporteur de la dopamine dans l'étape (c), dans lequel une disponibilité inférieure du transporteur de la dopamine dans l'étape (c) indique que le traitement est efficace.

26. Procédé selon la revendication 18, comprenant en outre l'administration au patient d'un ligand marqué du transporteur de la dopamine avant au moins une des étapes d'évaluation, les étapes d'évaluation comprennent l'évaluation du potentiel de liaison du transporteur de la dopamine, et l'étape de comparaison comprend la comparaison du potentiel de liaison du transporteur de la dopamine dans l'étape (a) au potentiel de liaison du transporteur de la dopamine dans l'étape (c), dans lequel un potentiel de liaison inférieur du transporteur de la dopamine dans l'étape (c) indique que le traitement est efficace.

27. Procédé de détermination du fait qu'un individu présente une plus grande prédisposition au TDAH, le procédé comprenant :
- la fourniture d'un agent d'imagerie du transporteur de la dopamine ;
- l'évaluation du transporteur de la dopamine dans au moins une région du système nerveux central dudit patient en utilisant ledit agent d'imagerie du transporteur de la dopamine ;
- la comparaison du niveau de transporteur de la dopamine chez ledit patient à un niveau de transporteur de la dopamine normal, dans lequel un niveau supérieur à un niveau normal indique une plus grande prédisposition au TDAH.

28. Procédé selon la revendication 27, comprenant en outre l'administration au patient d'un ligand marqué du transporteur de la dopamine avant l'étape d'évaluation, et dans lequel l'évaluation comprend la détermination de la quantité de ligand marqué du transporteur de la dopamine qui est lié au transporteur de la dopamine.

29. Procédé selon la revendication 28, dans lequel le ligand du transporteur de la dopamine comprend un composé qui se lie au transporteur de la dopamine.

30. Procédé de surveillance de l'évolution d'un traitement du TDAH chez un patient humain, le procédé comprenant :
- la fourniture d'un agent d'imagerie du transporteur de la dopamine ;
- en utilisant ledit agent d'imagerie du transporteur de la dopamine, l'évaluation du transporteur de la dopamine dans au moins une région du système nerveux central dudit patient plusieurs fois durant ledit traitement.

31. Procédé selon la revendication 30, comprenant en outre l'administration au patient d'un ligand marqué du transporteur de la dopamine avant l'étape d'évaluation, et dans lequel l'évaluation comprend la détermination de la quantité de ligand marqué du transporteur de la dopamine qui est lié au transporteur de la dopamine.

32. Procédé selon la revendication 31, dans lequel le ligand du transporteur de la dopamine comprend un composé qui se lie au transporteur de la dopamine.

33. Procédé selon la revendication 30, dans lequel le traitement comprend un traitement pharmaceutique.

34. Procédé selon la revendication 33, dans lequel le traitement pharmaceutique comprend l'administration d'un produit pharmaceutique choisi dans le groupe constitué par le méthylphénidate, la pémoline, et une amphétamine.

35. Procédé selon la revendication 34, dans lequel l'étape d'évaluation comprend l'imagerie PET ou SPECT.

36. Procédé selon l'une quelconque des revendications 1, 16, 17, 18, 19, 21, 25, 26, 27, 28, 30 et 31, dans lequel l'évaluation est non invasive pour le corps humain.
